(19) Europäisches Patentamt
European Patent Office
Office européen des brevets

(11) Publication number: **0 394 968**
**A1**

(12) **EUROPEAN PATENT APPLICATION**

(21) Application number: **90107780.0**

(51) Int. Cl.[5]: **C07C 209/26, C07C 209/52**

(22) Date of filing: **24.04.90**

(30) Priority: **25.04.89 US 342811**
**16.04.90 US 508003**

(43) Date of publication of application:
**31.10.90 Bulletin 90/44**

(84) Designated Contracting States:
**AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Dombek, Bernard Duane**
**1620 Woodvale Drive**
**Charleston, West Virginia 25314(US)**
Inventor: **Wenzel, Timothy Todd**
**618 Beech Avenue**
**Charleston, West Virginia 25302(US)**

(74) Representative: **von Hellfeld, Axel, Dipl.-Phys. Dr. et al**
**Wuesthoff & Wuesthoff Patent- und Rechtsanwälte Schweigerstrasse 2**
**D-8000 München 90(DE)**

(54) **Amination of carbonyls.**

(57) Organic compounds containing at least one carbonyl group are reductively aminated to minimize the formation of hydroxylated side product by the use of at least one of (i) an amination promoter, (ii) water removal, (iii) lower hydrogen partial pressure during the formation of an imine or enamine intermediate or (iv) primary or secondary amine reactant to form an imine or enamine intermediate.

EP 0 394 968 A1

# AMINATION OF CARBONYLS

This is a continuation-in-part of United States patent application Serial No. 342,811, filed April 25, 1989.

This invention pertains to processes for the reductive amination of at least one carbonyl group contained in organic compounds to an amine group, which processes can be conducted with the minimization of hydrogenation of the carbonyl group to a hydroxyl group. Moreover, preferred aspects of this invention pertain to processes which exhibit advantageous rates to the aminated compounds.

## Background to the Invention

The reductive amination of carbonyl-containing organic compounds has been proposed by numerous researchers.

Schmitt, et al., in U.S. Patent No. 3,352,913, which is based upon the same priority document as British Patent Specification 972,010, published October 7, 1964, disclose hydrogenating isophoronenitrile in the presence of catalysts

"especially those containing iron, cobalt, nickel, palladium and platinum and other Group VIII elements, such as ruthenium, rhodium, osmium, iridium, on supports such as diatomaceous earth, bentonite, montmorillonite, γ-alumina, kieselguhr, activated coal etc., with additives such as copper, chromium, thorium, etc. as desired." (Column 2, lines 17 to 23)

The process is said to be conducted at a temperature between 50° to 150° C, and the patentees caution that "...it is best to see to it that partial pressure of the hydrogen does not fall below about 50 atmospheres." The production of the diamine is favored by a proportion of 10 to 30 moles of ammonia per mole of ketonitrile.

The patentees note that:

"it has been known that ketonitriles can be hydrogenated, but in the particular case of the γ-ketonitriles they cannot be hydrogenated because they have a much greater tendency than the other ketonitriles to evolve hydrogen cyanide. Under the special conditions of the process of the invention, however, such phenomenon can be avoided." (Column 2, lines 62 to 68)

In essence, to prevent, or reduce, evolution of hydrogen cyanide, the amination process involved the use of very high hydrogen partial pressures, e.g., 50 atmospheres or more. Indeed, in the examples illustrating the amination of ketonitriles, Schmitt, et al., used hydrogen overpressures of 120 to 150 atmospheres. Experimental work using lower hydrogen partial pressures has confirmed the evolution of hydrogen cyanide from isophoronenitrile and a reduction in yield of the corresponding diamine. Moreover, the hydrogen cyanide appears to poison the hydrogenation catalyst.

Schmitt, et al., broadly state that:

"The process can be performed expediently in the presence of solvents, particularly the alcohols, such as methyl alcohol, ethyl alcohol, etc., and ether, but also hydrocarbons in general such as cyclohexane or the like." (Column 2, lines 69 to 72).

The only solvent explicitly demonstrated by the patentees is methanol.

Considerable efforts have been devoted to producing diamines from ketonitriles with desirable selectivities to the diamine. Additional efforts have been directed to achieving these results without the need for such high pressures.

Japanese patent application Kokai 62/123154, published November 25, 1985 (Daicel) is directed to the use of a Raney cobalt catalyst containing manganese as an additional component, which catalyst is used for the manufacture of isophoronediamine. The patent application notes that in the prior art, the reductive amination of isophoronenitrile was conducted at extremely high hydrogen pressures (120 to 150 atmospheres) with pulverized catalyst, and even then the yields to the isophoronediamine were low, e.g., 81.4%. The Japanese patent applicant asserts that with the specified catalyst, milder reaction conditions may be used. The examples report obtaining 83.3 to 89.6 percent yields of the isophoronediamine in the reaction solution with 3.9 to 5.6 percent of the 3-cyano-3,5,5-trimethylcyclohexanol hydrogenation product. While other changes exist, the hydrogen partial pressure is increased from 70 $kg/cm^2$ in Example 2 to 100 $kg/cm^2$ in Example 3. The yield of isophoronediamine in the crude reaction solution increased from 83.3 percent to 87.5 percent even though the ratio of catalyst to isophoronenitrile decreased. Hence, hydrogen partial pressure still appears to play a significant role in achieving the sought diamine product.

The Japanese patent application indicates that alcohols and glycols containing 1 to 4 carbon atoms (e.g., methanol, ethanol, ethylene glycol, etc.) can be appropriately used. Only methanol is used as the

solvent in the working examples.

West German patent application 3,011,656 discloses a continuous process for making isophoronediamine from isophoronenitrile by reacting the nitrile with ammonia at a temperature of about 40° to 100°C in the absence of catalyst to form 3-cyano-3,5,5-trimethylcyclohexyl imine, and then hydrogenating the imine in the presence of cobalt-, nickel- or iron-containing catalyst to form the diamine. The patent application states that the hydrogen concentration during the formation of the imine should not exceed 0.05 weight percent. The example indicates that the process is conducted at a pressure of about 300 bar.

U.S. Patent No. 4,429,157 discloses a process for making amines from, e.g., isophoronenitrile by reacting the keto group with ammonia in the presence of an imine-forming catalyst which is an ion exchanger loaded with ammonium ion, and then subjecting the nitrile-containing imine to hydrogenation in the presence of ammonia and hydrogen in the presence of a hydrogenation catalyst. The patentees state that the imine-forming reaction may be conducted at 10° to 120°C under autogenous or elevated pressure, e.g., up to 300 bar. The amination is then conducted at a temperature of 80° to 200°C and elevated pressure, i.e., 80 to 300 bar.

U.S. Patent No. 2,292,949 discloses processes for catalytic hydrogenation of 2-iminonitriles. The patentees state that the hydrogen partial pressure for the hydrogenation should be at least about 10 atmospheres with the total pressure within the range of 1500 to 3000 pounds per square inch and the temperature is between about 50° to 170°C, preferably between 100° and 150°C. Hydrogen is added from time to time, if necessary, to maintain the total pressure within the working range. The hydrogenation is preferably conducted using a nickel or a cobalt catalyst. Anhydrous ammonia is present to "stabilize the imino group and minimize condensation" (column 2, lines 38 and 39). The patentees state at column 2, line 45 to column 3, line 7:

"Other solvents are not essential for the successful operation of the process, although in many instances the use of an organic solvent has been found beneficial in improving yields and reducing the conversion to degradation and other by-products. Hydrocarbons, alcohols, and ethers are among the preferred solvents as exemplified by toluene, methanol, and dioxane. These materials are but typical examples, and a wide variety of other solvents of the same general class can be used with good effect. Generally speaking, if a solvent is employed, it is preferred to use methanol, owing to its low cost, freedom from catalyst poisons, excellent solvent action on both the starting materials and products, and ease with which it is separated from the crude hydrogenation mixtures without introducing needless product losses."

U.S. Patent No. 3,544,485 discloses a method for activating Raney alloy catalysts for hydrogenation. The activation is conducted in the presence of water and one of aluminas, amines, non-N-substituted lactams, N-substituted lactams, oximes, N-substituted acid amides and urea derivatives. The patentees suggest that the hydrogenation and activation can occur simultaneously. In Table III, Experiments 15, 16, 17, 21, 23, 26, 29, 32, 39, 43, 44, 46, 50, 54, 57 and 62 involve hydrogenation of nitriles to amines. Experiments 35 and 39 involve hydrogenating cyclohexanoneoxime to cyclohexylamine.

Without wishing to be limited to theory, it is believed that the amination of a carbonyl group proceeds through conversion of the carbonyl group to an imino group which is then hydrogenated to form the amine. The carbonyl group and the imino group are believed to be in equilibrium. Thus, during the reduction amination, both carbonyl and imino species will be present. The carbonyl group is also subject to being hydrogenated to form a hydroxyl group. Dehydrogenation of the hydroxyl group under reaction conditions is often difficult and, therefore, an unwanted side product, i.e., a hydroxylated compound, may be substantially irreversibly generated.

Accordingly, processes are sought which minimize the formation of the hydroxyl-containing side product, yet still provide advantageous rates to the aminated product.

## Summary of the Invention

By this invention processes are provided for aminating a feed comprising organic compound containing at least one carbonyl group without undue generation of hydroxylated compounds through the hydrogenation of the carbonyl group. In accordance with the processes of this invention, the carbonyl-containing organic compound contained in liquid menstruum is subjected to reductive amination conditions in the presence of reductive amination catalyst to produce an amine product under conditions which enhance selectivity to the amine product as compared to a hydroxylated product. Preferably, the amination is conducted with a hydrogen partial pressure less than about 1000 psi (pounds per square inch) (68 bar), e.g., less than about 700 psi (48 bar), say, about 100 to 700 psi (7 to 48 bar), and sometimes about 100 to

500 psi (7 to 34 bar) to further minimize hydrogenation of the carbonyl group to form a hydroxyl group.

In one aspect of the invention, the liquid menstruum comprises a sufficient amount of at least one amination promoter to enhance the selectivity of the reductive amination to the aminated product as compared to the hydroxylated product. The amination promoters are dipolar protic compounds, preferably organic, especially oxygenated organic compounds, having more than one carbon atom. The preferred amination promoters have high dielectric constants, e.g., at least about 8, preferably at least about 15, to 25° C, and exhibit a $PK_a$ at 25° C in a 0.4 to 1 weight per cent solution in dimethyl sulfoxide of less than about 35. Preferred amination promoters contain at least one hydroxyl group. The amination promoters may essentially comprise the solvent for the amination and generally at least about 1, say, at least about 5, e.g., about 10 to essentially 100, or even about 20 to essentially 100, weight percent of the solvent is amination promoter. Often, the mole ratio of amination promoter to carbonyl group is at least about 0.1:1, e.g., 0.1:1 to 100:1 or more, e.g., about 0.5:1 to 70:1.

In yet another aspect of this invention, a primary amine or secondary amine is used to form an imine or enamine under imine-forming conditions which then is hydrogenated in the presence of hydrogen or reductively aminated in the presence of hydrogen and ammonia (wherein the ammonia equilibrates with the imino or enamino group) to form the amine. Advantageously, when imines or enamines are prepared from primary or secondary amines, enhanced selectivities to aminated products as opposed to hydroxylated products tend to occur.

In another aspect of this invention, the carbonyl group is converted to an imino or enamino group by reaction with ammonia or primary amine to produce an imino group or with secondary amine to produce an enamino group and then the imino or enamino group is converted to the amine wherein the hydrogen partial pressure during at least a portion of the imine-forming or enamine-forming step is at least 100 psi (7 bar) lower than the hydrogen partial pressure during at least a portion of the step in which the imino or enamino group is converted to an amino group.

In a further aspect of this invention, the carbonyl is converted to an imine or enamine. Because the imine or enamine is formed without the necessity of using ammonia, water can be removed during the reductive amination and, due to equilibria principles, favor the formation of the amine as opposed to the hydroxylated product. The removal of water may be by physical means such as by distillation, sorption (absorption or adsorption), phase separation or membrane permeation wherein the water is physically removed from the reaction zone. The removal may be effected through chemical or physiochemical interaction with components retained in the reaction medium, e.g., by absorption with liquid or solid components such as polar liquids such as alcohols or by adsorption on solid components such as molecular sieves. The primary or secondary amine to generate the imine or enamine may be solid or form a solid imine or enamine product or be substantially immiscible with water to facilitate water separation. The imine or enamine may then be converted to primary amine by equilibration with ammonia under reductive amination conditions or hydrogenated to form a secondary or tertiary amine.

In yet another aspect of this invention, reductive amination catalyst is activated by contact with hydrogen at a sufficient partial pressure and temperature for a time sufficient to increase reductive amination activity. Often, the hydrogen partial pressure for regeneration is at least about 500 psi (34 bar), say, 600 to 2000 psi (40 to 136 bar), and the temperature is at least about 40° C, say 50° to 150° C. The duration of the regeneration will vary depending upon the regeneration conditions and, if the activation is for the regeneration of a catalyst, the severity of the deactivation. Frequently, the regeneration is conducted for a period of about 1 to 50 or more hours. Preferably, the activation is conducted in the substantial absence of ammonia or amine.

## Detailed Discussion

Amino compounds find a wide range of utilities such as oil additives, dispersants, fabric treating agents, and intermediates to other useful compounds.

The carbonyl-containing compounds useful in this invention may be those containing at least one carbonyl group or those which are capable of generating at least one carbonyl group under reductive amination conditions. Exemplary of the compounds containing at least one carbonyl group include those represented by the formula $R^1C(O)R^2$ wherein $R^1$ may be hydrogen or a substituent represented by $R^2$ and wherein $R^2$ may be the same as or different from $R^1$ and is hydrocarbyl or substituted hydrocarbyl of 1 to 20 or 30 carbons. The hydrocarbyl may be alkyl, cycloalkyl, aryl, alkaryl, or aralkyl, and may be substituted with one or more substituents which do not adversely participate in the reductive amination. Representative substituents include aryl, aryloxy, alkyloxy, and nitrile moieties. $R^1$ and $R^2$ may join to form cyclic

structures.

One class of carbonyl-containing compounds is the carbonylnitriles represented by the structural formula

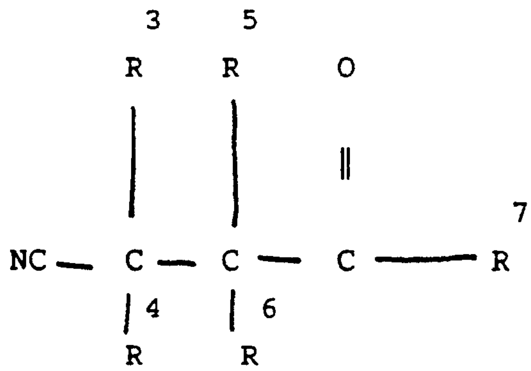

wherein $R^3$, $R^4$, $R^5$ and $R^6$ are the same or different and are selected from the group consisting of hydrogen, alkyl (e.g., 1 to 8 carbon atoms), cycloalkyl (e.g., 5 to 8 carbon atoms), aryl (e.g., monocyclic or bicyclic aryl of 6 to 12 carbon atoms), aralkyl (e.g., of 7 to 12 carbon atoms), and alkaryl (e.g., of 7 to 12 carbon atoms), wherein at least $R^3$ and $R^4$ are other than hydrogen; and $R^7$ is hydrogen, alkyl (e.g., 1 to 8 carbons), cycloalkyl (e.g., 5 to 8 carbon atoms), aryl (e.g., monocyclic or bicyclic of 6 to 12 carbon atoms), alkaryl (e.g., 7 to 12 carbon atoms), aralkyl (e.g., 7 to 12 carbon atoms) and $R^7$ may form a cyclic compound with a carbon atom of one of $R^3$ and $R^5$. Frequently, the nitrile group is on the beta carbon to the carbon atom of the carbonyl group or further removed therefrom, and advantages provided by this invention are often most realized when the nitrile group is on the beta carbon atom. Exemplary of the carbonylnitriles are isophoronenitrile, 4-methyl-5-cyanohexan-3-one, 4-methyl-4-cyanopentan-2-one, 2-methyl-3-cyanopentanal, 2-ethyl-3-cyanohexanal, 3,5-dicyano-3,5-dimethylcyclohexanone, 2,2,6,6-tetramethyl-4-ketopimelonitrile, 3-cyano-3-methylcyclohexanone, methyl isobutyl ketone, methylethyl ketone, isophorone, 2,6-dimethylcyclohexanone, butyraldehyde, 2-ethylhexanal, pivaldehyde, and pinacolone.

Compounds which are capable of providing at least one carbonyl group under reductive amination conditions include alcohols which are readily dehydrogenated; aromatic alcohols (e.g., phenol); 1,2-diketones; enols and vinyl alcohols.

The reductive amination of the carbonyl group is generally conducted at a temperature between about 10° to 300° C, preferably about 15° to 250° C. The temperature should not be so high as to deleteriously affect the reactants or products but should be sufficient to achieve an acceptable reaction rate. Thus, in many instances, the temperature is between about 50° and 200° C. The pressure may be autogeneous to elevated. Preferably elevated pressures are used. High pressures, e.g., 1500 psig or more, may be used. However, lower pressures are often suitable, e.g., less than about 700 psig, e.g., from about 100 to 500 psig. The temperature is below that which causes under the reaction conditions undue decomposition of the feed or intermediates or aminated product.

Hydrogen is also present and is generally in an amount of at least 5 moles per mole of carbonyl group, e.g., from about 5 to 1000 or more moles per mole of carbonyl group. The hydrogen is often used as the pressurizing gas in the reactor. Hence, the partial pressure of hydrogen is often about 50 to 1500 psig. Frequently, the partial pressure of hydrogen is at least about 30, say, about 50 to 95, percent of the total absolute pressure of the reaction system. Advantageously, the lower total pressures and the lower hydrogen partial pressures which may be used in accordance with the preferred aspects of this invention can provide enhanced selectivity to the aminated product as opposed to hydroxylated product.

In a preferred aspect of the processes of this invention, the hydrogen partial pressure is less during the portion of the reductive amination in which the carbonyl group (or precursor to the carbonyl group) is being converted to the imino (or enamino) group, and the hydrogen partial pressure is increased during the portion of the reaction in which the imino (or enamino) group is being converted to the amino group. Since most carbonyl groups and corresponding imino (and enamino) groups are thought to be in equilibrium, the practicality of achieving beneficial effects by varying the hydrogen partial pressure will depend upon the rapidity of interchange between the carbonyl and imino groups in the equilibrium and the extent of the equilibrium. When varying hydrogen partial pressures are used, the initial hydrogen partial pressure may be essentially 0 psi, and the peak hydrogen partial pressure may be at least about 100 psi (7 bar), say, about

100 to 1000 or more psi (7 to 68 bar) greater than the minimum hydrogen partial pressure during the reaction.

At least one of ammonia, primary amine and secondary amine is typically provided in an amount of at least 1.5 mole of -NHR per equivalent of carbonyl group, often about 5 to 50, preferably 10 to 30 moles of nitrogen source per mole of carbonyl group.

The nitrogen source for the formation of the imines may be ammonia and/or primary amine and for the formation of enamines may be secondary amines. The nitrogen sources which may be used to form imino and enamino groups have the formula $R^1R^2NH$ wherein $R^1$ and $R^2$ may be hydrogen, hydroxyl, alkyl, hydroxyalkyl, aminoalkyl, aryl, hydroxyaryl, aminoaryl, amino, and the like of up to 20 carbon atoms and $R^1$ and $R^2$ may join to form cyclic compounds such as piperazine. Examples of primary and secondary amines are methylamine, dimethylamine, ethylamine, diethylamine, propylamine, dipropylamine, ethylenediamine, benzylamine, piperazine, morpholine, ion exchange resins bearing amine groups, pyrazole, pyrazoline, methanolamine, dimethanolamine, ethanolamine, diethanolamine, and aminoethylethanolamine. The amine may conveniently be the product amine, e.g., isophoronediamine when isophoronenitrile is used as the starting material. In many instances, the use of a primary or secondary amine to form an imino or enamino intermediate tends to enhance the selectivity of the process to the aminated product as opposed to hydroxylated product. When the imino or enamino group is converted to the amino group, the presence of ammonia is preferred, and the ammonia may constitute at least about 50 mole percent, say, about 50 to 95 or virtually 100 mole percent, of the total ammonia and amine. When ammonia is present, the imino or enamino groups formed from amine may equilibrate and thus, a primary amine can result from the presence of ammonia during the conversion of the imino group or enamino group to the amino group. Alternatively, in the essential absence of ammonia, the imino group or enamino group may be hydrogenated to form a secondary or tertiary amino group. When the primary amine is the same as the ultimate aminated product, numerous advantages can be obtained including avoiding any separation of the primary amine from the aminated product.

The amination is conducted in the presence of a hydrogenation (reductive amination) catalyst. Hydrogenation catalysts comprise compounds and metals of Group VIII of the Periodic Table of the Elements, e.g., platinum group metals, as well as chromium, manganese, copper, zinc, molybdenum, tungsten, and rhenium and combinations such as copper chromite. Preferred catalysts comprise cobalt-, iron- or nickel-containing catalysts, especially Raney nickel and Raney cobalt catalysts. The Raney nickel and Raney cobalt catalysts may contain additional elements to enhance activity or selectivity. These promoting elements (which may be present in elemental or chemically-combined form) include manganese, chromium and the like. The most preferred catalysts are Raney cobalt catalysts, especially those containing 0.01 to 10 weight percent manganese and/or chromium.

Those catalysts capable of being supported may be placed on suitable carriers such as alumina, carbon, kieselguhr, bentonite, asbestos, silica, titania, zirconia, etc. The active compounds may be provided in an amount of 0.5 to 50 weight percent of the total supported catalyst.

The amount of catalyst provided will depend upon the activity of the catalyst and the form of the catalyst and the type of reactor used. In general for slurry reactors, the catalyst is provided in an amount of at least about 0.02 gram per gram of the carbonyl-containing compound, e.g., often about 0.05 to 2, preferably 0.1 to 1, grams per gram of the carbonyl-containing compound.

The reaction may be conducted in a batch, semicontinuous or continuous reactor, and the formation of the imino group and the formation of the amino group may be conducted in the same or different reaction zones and may be conducted simultaneously or sequentially. In the semi-continuous mode, a preferred operation is by continuously or periodically adding the carbonyl-containing compound to the reaction medium. The catalyst may be a fixed bed catalyst or may be in the form of a slurry. Homogeneous catalysts may also be used. Slurry reaction systems are generally preferred with Raney nickel and Raney cobalt catalysts. Raney cobalt catalysts are often the most preferred due to their tendency to produce less hydroxylated side products.

The reaction time for aminating the carbonyl group is preferably sufficient to enable at least about 90 percent, preferably at least about 95 percent, preferably essentially all of the carbonyl groups to be consumed. Frequently, the reaction is conducted until the hydrogen up-take rate becomes very slow. Often, the reaction time is at least about 0.01 hour, e.g., about 0.05 to 50, say, about 0.1 to 5 hours.

The reaction is conducted in the presence of a suitable solvent, i.e., one which is substantially inert in respect of being aminated or reacting with the feed or intermediates or product under the reaction conditions. Solvents include hydrocarbons including aliphatic and aromatic hydrocarbons such as butane, isobutane, pentane, cyclopentane, hexane, cyclohexane, isopentane, benzene, ethylbenzene, xylene, toluene, etc.; alcohols containing 1 to about 6 carbon atoms such as methanol, ethanol, n-propanol,

isopropanol, n-butanol, isobutanol, t-butanol, n-pentanol, 2-pentanol, 3-pentanol, hexanol, cyclohexanol, phenol, etc.; tertiary amines such as triethanolamine, triethylamine, diethanolethylamine, etc.; pyridine; piperazine; morpholine; diols of 1 to about 6 carbon atoms such as ethylene glycol, propylene glycol, 1,3-propanediol, 1,4-butanediol, 1,2-butanediol and 2,3-butanediol; and ethers of 1 to about 6 carbon atoms such as dimethylether, diethylether, methylethylether, diethylene glycol, diethylene glycol monomethyl ether, propylene glycol monomethyl ether, ethylene glycol dimethyl ether, triethylene glycol dimethyl ether, Cryptands, triethylene glycol, tetraethylene glycol, tetrahydrofuran, etc. The solvents containing hydroxy groups can react to some extent during the reductive amination; however, this reaction is typically minimal, especially with the hydroxyl group being on a primary or secondary carbon atom. Preferably, the solvent does not contain components, or is not readily degenerated or reacted into components under reductive amination conditions, that are deleterious to the catalyst such as carbon monoxide, sulfur, halide, hydrogen cyanide, etc. Thus, often, the solvents have more than one carbon atom and have an essential absence of sulfur, halide and nitrile groups. Advantageously, the solvent provides a reaction mixture at 25° C having a viscosity of less than about 50, preferably less than about 25, centipoise. When a solvent is used, it is often provided in a weight ratio to the carbonyl-containing compound of at least about 0.5:1, say, about 0.5:1 to 20:1.

The nature and amount of the solvent should not be such that the catalyst becomes unduly deactivated during the amination under the conditions of the amination. Methanol has been proposed as the solvent in the amination of, e.g., carbonyl-containing compounds at high pressures. At lower pressures, especially at pressures below about 700 psig as are preferred in accordance with this invention, catalysts deactivate rapidly with methanol as the sole solvent. At the higher hydrogen partial pressures, e.g., above about 1500 psig, the deactivation rate in a methanol menstruum is typically substantially reduced.

As can be perceived from the foregoing discussion, a wide variety of reaction sequences can be used while still obtaining the benefits of the processes of this invention. For instance, the processes may be conducted in a single step or may proceed stepwise under the same or differing conditions and intermediate products may be isolated or remain in the reaction menstruum for subsequent reaction to form the sought amine.

In an aspect of the invention, the feed containing the organic compound with at least one carbonyl group is contacted with at least one of ammonia, primary amine and secondary amine under imine-forming conditions to convert at least one carbonyl group of the organic compound in at least a portion of the feed to an imino or enamino group (unsaturated nitrogen group) to provide an imine or enamine (unsaturated nitrogen compound) product and at least a portion of the imine or enamine product is contacted with hydrogen under amine-forming conditions to convert at least one imino or enamino group of at least a portion of the imine or enamine product to an amine group to produce an amine product. The formation of the unsaturated nitrogen group may be simultaneous with at least a portion of the amine-forming step, or separate stages may be used with intermediate recovery of the unsaturated nitrogen compound.

The imine-forming conditions (which are intended herein to include enamine-forming conditions) may be the same or different than those used in the amination of the unsaturated nitrogen group, and broadly fall within the reductive amination conditions described herein. Advantageously, the conditions may be selected to enhance formation of the unsaturated nitrogen group and minimize the potential for the conversion of the carbonyl group to a hydroxyl group. Thus, catalysts, temperatures, hydrogen partial pressures, and the like may differ from those used in the amination of the unsaturated nitrogen group. Catalysts may not be required, especially when using an amination promoter. When catalysts are present, they can include the hydrogenation catalysts described above and also include ion exchange resins, aluminas (e.g., alpha, gamma, beta and other transition aluminas), titania, zirconia, magnesium aluminates (including spinels), takovites and hydrotalcites, molecular sieves including aluminosilicates, silicoaluminophosphates, titanium silicates, and titanium-alumino-silicates, etc.

In a preferred aspect of the invention, hydrogen is not present, or is present in an amount less than that present, during the amination step. Thus, the hydrogen partial pressure is often at least about 100 psi (7 bar) less during at least a portion of the step forming the unsaturated nitrogen group than that during at least a portion of the amination step. Often, the unsaturated nitrogen forming step is conducted under a hydrogen partial pressure of 0 to about 500 or 700 psi (about 0 to 34 or 48 bar).

The amine-forming conditions also fall broadly within the reductive amination conditions disclosed herein. The catalyst is preferably a hydrogenation catalyst as disclosed above. The hydrogen partial pressure is also preferably within the ranges set forth for the reductive amination conditions. The amine-forming conditions do not require the presence of nitrogen source, i.e., at least one of ammonia, primary amine and secondary amine although the presence of ammonia is preferred when primary amines are sought. Thus, the amine may be formed by hydrogenation. When a primary or secondary amine is used as

the nitrogen source for the formation of the imine or enamine, a secondary or tertiary amine results. Often, the primary amine is sought as an intermediate to other products such as isocyanates. Even so, advantages can be obtained through using a primary amine or secondary amine to generate the imino or enamino groups. For instance, the imino or enamino groups may be capable of being separated from the reaction mixture or otherwise do not participate extensively in the equilibrium with the carbonyl group, thus shifting the equilibrium to favor the imino or enamino group over the carbonyl group. Also, the steric hindrance provided by the larger nitrogenous group can affect the rate of reversion to the carbonyl group. A particularly attractive advantage is that water, a by-product of the reaction with the carbonyl group, can be more readily separated from the reaction menstruum than when ammonia is the nitrogen source and water must be separated from ammonia. Removal of the water will shift the carbonyl/unsaturated nitrogen group equilibrium in favor of the unsaturated nitrogen group. The primary amine can be generated from the unsaturated nitrogen compound by reequilibrating during the amination with ammonia.

In accordance with a preferred aspect of the invention, amination promoter, which may comprise solvent, is provided in the reacton menstruum during the reductive amination. When an amination promoter is used, it is preferably present during at least a portion of the imine or enamine forming step. The amination promoters are dipolar protic compounds and are preferably liquid at reaction conditions. Generally, the amination promoters exhibit a $pK_a$ at 25° C in a 0.4 to 1 weight per cent solution in dimethylsulfoxide less than about 35, preferably, less than about 30, often, about 1 to 30. The procedure is disclosed in Matthews, et al., J. Am. Chem. Soc., vol 97, pp 7006 to 7014 (1975), herein incorporated by reference. Amination promoters having lower $pK_a$ values are frequently used in lower concentrations to avoid adversely affecting the catalyst. Often the amination promoters have a dielectric constant of at least about 8, preferably at least about 15, at 25° C and sometimes a dipole moment greater than about 1 Debye. The preferred amination promoters have more than one carbon atom. Preferred promoters include compounds containing at least one, preferably at least two or more, hydroxyl groups such as ethylene glycol; 1,2-propylene glycol; 1,3-propanediol; 1,2-butanediol and 1,4-butanediol. Advantageous promoters include monoalcohols, diols, triols and polyols, especially having a molecular weight of less than about 200. Promoters include methanol, ethanol, n-propanol, iso-propanol, n-butanol, iso-butanol, t-butanol, ethylene glycol, 1,2-propylene glycol, 1,3-propanediol, 1,2-butanediol, 1,3-butanediol, 1,4-butanediol, 2,3-butanediol, glycerol, diethylene glycol, triethylene glycol, tetraethylene glycol, sorbitol, pentanediols, hexanediols, diethylmonoethanolamine, diethanolmonoethylamine, triethanolamine, phenols, piperazine, morpholine, hydroxyethylethylene urea, hydroxyesters, hydroxyamides, amides and the like. Preferably, the promoter does not contain reactive hydrogens such as on primary and secondary amines and is devoid of groups which can be hydrogenated or aminated under reaction conditions such as carbonyl, imino, and nitrile groups. Desirably, the amination promoter does not contain certain components, or is not readily degenerated or converted under reaction conditions to components, that are deleterious to the catalyst such as carbon monoxide, sulfur, halides, hydrogen cyanide and the like.

The amount of promoter that is present may vary widely, e.g., up to the total amount of solvent present when the solvent comprises promoter. Often, the amount of promoter is present in a mole ratio to the nitrile to be aminated of at least about 0.05:1, preferably at least about 0.1:1. say, about 0.1:1 to 20:1 or more. Preferably, the amount of promoter does not unduly increase the viscosity of the reaction menstruum which preferably has a viscosity of less than about 25 centipoise at 25° C.

Advantageously, monoalcohols and diols having 1 to 3 carbon atoms are used in conjunction with another solvent. In some instances, these lower molecular weight alcohols can deactivate or otherwise adversely affect the catalyst, especially at lower hydrogen partial pressures, e.g., below about 1000 psig, say, below about 700 psig. Also, ethylene glycol and other diol or polyol-containing promoters can lead to undesirable viscosities and/or foaming on depressurization. Further, some promoters can tend to promote, or allow to occur, condensation reactions between nitriles and amines. This disadvantage can often be eliminated or limited by using (i) combinations of promoters and solvents other than promoters or (ii) combinations of promoters having deleterious side effects with those promoters not exhibiting the undesirable side effects, yet the benefit provided by the promoters may still, to a substantial degree, be obtained. Often, the weight ratio of promoter to total solvent and promoter is at least about 0.01:1, say, at least about 0.1:1, e.g., 0.1:1 to 0.9:1, preferably, about 0.1:1 to 0.8:1.

## Examples 1 to 9

All reactions are performed in 100 ml T316 stainless steel or Hastelloy C-276 Parr, electrically heated

autoclaves equipped with a magnetically driven stirrer driven at maximum speed. Samples are periodically withdrawn through a decanter tube positioned about 8 millimeters above the bottom of the reactor. Isophoronenitrile (IPN) is sublimed before use (78 °C, $10^{-4}$ torr). Raney catalysts are washed with water, and then methanol several times, before storing under methanol. Weights of catalyst reported are solvent-wet weights. All other materials are used as received.

A. Batch reactions are carried out by charging methanol, catalyst, isophoronenitrile and a tetrahydronaphthalene internal standard to the autoclave bottom. The bottom is attached to the autoclave head, along with a stainless steel cylinder filled with the required amount of ammonia. The entire apparatus is purged of air by briefly evacuating, and then filling with hydrogen to 50 psig two times. After venting to atmospheric pressure, the ammonia is added causing a brief exotherm. The reactor is then heated to the temperature set forth in Table I below, and then immediately pressurized with hydrogen. Hydrogen uptake is monitored by timing pressure drops. Reaction progress is monitored by occasionally removing samples via the decanter tube, which are analyzed by capillary gas chromatography. These samples showed essentially complete conversion of isophoronenitrile to the aminonitrile intermediate after completion of the first stage of the reaction. When hydrogen uptake slowed, or after 1 hour at the initial temperature, the mixture is brought to the final temperature in steps of about 20 °C, holding at these intermediate temperatures for about 30 minutes to 1 hour. Final temperature is held for about 30 minutes to 2 hours. In example 5, the initial 80 °C temperature is maintained for one hour, an intermediate temperature of 100 °C is maintained for one hour and the final temperature at 120 °C is maintained for one hour. In example 6, the initial temperature of 70 °C is maintained for 0.5 hour, and the temperature is thereafter increased at a rate of 5 °C per 15 minutes until the final temperature of 120 °C is achieved and the final temperature is maintained for one hour.

B. Semicontinuous process reactions involve the addition of ammonia-pressurized solutions of isophoronenitrile. Catalyst is charged as a slurry in 15 milliliters of methanol to the reactor bottom and deoxygenated as described above. The isophoronenitrile is dissolved in sufficient methanol to make a 40 weight percent solution and charged, along with the tetrahydronaphthalene internal standard, to a glass, graduated Fischer-Porter bottle. Ammonia is introduced into this solution via a 1/8" tube inserted to near the bottom of the Fischer-Porter bottle. All of the ammonia for the reaction is charged in this way, with none charged to the reactor itself. After achieving the sought pressure and temperature in the Parr autoclave, the ammonia-pressurized isophoronenitrile solution is fed into the autoclave during 2 hours using a high pressure pump. Aliquots are periodically removed to monitor the reaction progress. After complete addition, the autoclave is brought to the final temperature in 20 ° increments as before. In example 9, the final temperature of 120 °C is maintained for 0.5 hour.

Examples 1, 2, 3, 4 and 8 are comparative. In examples 1, 2, 3 and 8, no final temperature increase is effected. The reaction conditions and results are summarized in Table I.

TABLE I

| | | | | | | | | temperature (°C) | | % Yield | | | |
|---|---|---|---|---|---|---|---|---|---|---|---|---|---|
| Example | Mode | Catalyst | g IPN | g catalyst | g $NH_3$ | mL MeOH | psi | initial | final | IPDA[a] | bicyclic[b] | IPANI[c] | IPAA[d] |
| 1* | batch | RaCo[e] | 6.0 | 2.01 | 4.60 | 34 | 1200 | 100 | 100 | <2% | <2% | 5% | <2% |
| 2* | batch | RaCo | 6.03 | 4.02 | 5.03 | 34 | 1475 | 100 | 100 | 89% | 3% | <1% | 5% |
| 3* | batch | RaCo-Cr[f] | 6.01 | 1.52 | 5.70 | 34 | 1200 | 100 | 100 | 89% | 5% | 1% | 6% |
| 4* | batch | RaCo-Cr | 6.01 | 0.61 | 5.24 | 34 | 1200 | 100 | 100[g] | <2% | <2% | <2% | <2% |
| 5 | batch | RaCo-Cr | 6.01 | 0.61 | 4.87 | 34 | 1200 | 80 | 120 | 79% | 6% | 6% | 8% |
| 6 | batch | RaCo-Cr | 6.02 | 1.55 | 5.20 | 34 | 500 | 70 | 120 | 85% | 7% | 3% | 5% |
| 7 | batch | RaCo-Cr | 8.03 | 2.08 | 5.00 | 45 | 250 | 50 | 120 | 69% | 7% | 15% | 2% |
| 8* | semi | RaCo-Cr | 15.02 | 1.54 | 12.38 | 44 | 1200 | 120 | 120 | <2% | <2% | <2% | <2% |
| 9 | semi | RaCo-Cr | 15.19 | 1.51 | 12.50 | 44 | 1200 | 85 | 120 | 82% | 5% | 8% | 4% |

* comparative

[a] isophoronediamine

[b] 1,3,3-Trimethyl-6-azabicyclo [3.2.1] octane

[c] stereoisomer of 3-cyano-3,5,5-trimethyl-aminocyclohexane which is less reactive

[d] 3-cyano-3,5,5-trimethyl cyclohexanol (both cis and trans steroisomers)

[e] Raney cobalt catalyst

[f] Raney cobalt catalyst available as Raney 2724 from Davison Chemical Div., W. R. Grace & Co., Baltimore, Maryland, U.S.A.

[g] The temperature is then increased to 120°C but no further reaction is perceived.

## Examples 10 to 51

The reactions are conducted in a 100 milliliter Parr minireactor which is electrically heated and equipped with a magnetically driven stirrer. In a dry box, 5 grams of dry, chromium promoted Raney cobalt (available as Raney 2724 from Davison Chemical Div., W. R. Grace & Co., Baltimore, Maryland) is placed into the minireactor. The minireactor is sealed with a rubber stopper and septum and removed from the dry box. Thirty-five milliliters of the solvent identified in Table II, which solvent has been sparged with hydrogen, are added to the minireactor by syringe through the septum. (When mixtures of solvents are used, the ratios set forth in Table II are by weight.) Then the stopper is removed for only a time sufficient to add about 12.1 grams of isophoronenitrile (see Table II) and 0.6 gram of triglyme (triethylene glycol dimethyl ether). The minireactor is then sealed, purged of air by three pressure/vent cycles with 200 psig hydrogen and thereafter pressure tested with 500 psig hydrogen for five minutes. The minireactor is again vented, and 9.9 grams of ammonia are added. The temperature of the reaction is brought to 60 °C and the slurry stirred at moderate speed for one hour. Hydrogen is then added to raise the pressure to 625 psig and the stirring is increased to the maximum rate. The time required for the pressure to drop 50 psi is observed and is reported in Table II. The pressure is increased to 625 psig and the time for the following 50 psig drop is observed and reported in Table II. After 80 minutes, the temperature is increased in 20 °C increments every 20 minutes until 130 °C is obtained (1 hour and 10 minutes) and the reactor is then held at 130 °C for 1 hour. The reactor is cooled and the contents analyzed. The results are summarized in Table II.

As indicated in Table II, several catalysts are used for more than one run. When the catalyst is used for more than one run, the supernatant liquid is withdrawn, and, usually, the catalyst is washed several times with 50 milliliter aliquots of the solvent (which has been sparged with hydrogen) while maintaining the minireactor at about 5 to 10 psig. About 10 milliliters of solvent are retained with the catalyst when the supernatant liquid is removed. The isophoronenitrile in about 25 milliliters of solvent which has been saturated with ammonia is added via the septum. The runs are otherwise essentially conducted as above.

The results summarized in Table II are normalized to 100% based on the components reported. In the table, the following terms have the following meanings:

MeOH: methanol
EtOH: ethanol
AmberlystTM 15: a styrene-divinyl benzene ion exchange resin available from Rohm & Haas, Philadelphia, PA.
t-BuOH: t-butanol
Me CarbitolTM: diethylene glycol monomethylether
THF: tetrahydrofuran
Methyl PropasolTM: 1-methoxy-2-propanol
Et glycol: ethylene glycol
Hexylene glycol: 2-methyl-2,4-pentanediol
Pr glycol: propylene glycol
1 PrOH: isopropanol
amide: 1-amino-3,5,5-trimethyl-3-cyclohexylamide
bicyclic, IPANI, IPAA: as defined in the previous examples.

TABLE II

| example | solvent | gIPN | 50 psi drop 1 (min) | 50 psi drop 2 (min) | % yield bicyclic | IPAN1 | IPDA | IPAA | amide |
|---|---|---|---|---|---|---|---|---|---|
| 38 | 2:1 t-BuOH:Pr glycol | 12.1 | 0:03:16 | 0:03:28 | 7.21% | 0.15% | 86.80% | 4.25% | 1.33% |
| 39 | 1:2 t-BuOH:Pr glycol | 12.2 | 0:03:49 | 0:03:29 | 6.43% | 0.09% | 89.48% | 2.69% | 0.85% |
| 40 | 9:1 t-BuOH-Pr glycol | 12.2 | 0:05:14 | 0:04:50 | 4.48% | 0.07% | 67.77% | 21.88% | 5.45% |
| 41 | 1:2 Pr glycol:tetra ethylene glycol | 12.0 | 0:06:47 | 0:05:20 | 3.68% | 0.03% | 76.03% | 15.10% | 4.61% |
| 42 | 9:1 t-BuOH:sorbitol | 12.2 | 0:11:52 | 0:13:00 | 6.02% | 10.89% | 74.25% | 7.55% | 0.88% |
| 43 | 9:1 t-BuOH:glycerol | 12.1 | 0:04:55 | 0:07:57 | 5.41% | 0.88% | 81.40% | 11.12% | 1.05% |
| 44 | 2:1 EtOH:Pr glycol | 12.0 | 0.02:51 | 0:02:48 | 5.56% | 0.11% | 89.25% | 3.32% | 1.36% |
| 45 | 1:2 EtOH:Pr glycol | 12.0 | 0:03:19 | 0:03:13 | 5.89% | 0.00% | 89.99% | 2.87% | 0.79% |
| 46 | 1:2 EtOH:tetraglycol | 12.0 | 0:07:14 | 0:05:15 | 4.02% | 0.12% | 83.43% | 7.62% | 4.10% |
| 47 | 1:2 1PrOH: Prop Glycol | 12.1 | 0:04:01 | 0:03:59 | 6.22% | 0.09% | 89.53% | 2.96% | 0.71% |
| 48 | 2:1 1PrOH:Pr glycol | 12.1 | 0.03:24 | 0:03:33 | 5.38% | 0.14% | 85.08% | 6.83% | 2.16% |
| 49 | 1:2 Me Carbitol:Pr glycol | 12.1 | 0:03:45 | 0:03:37 | 6.07% | 0.10% | 89.58% | 2.97% | 0.80% |
| 50 | 2:1 Me Carbitol:Pr glycol | 12.1 | 0.03:48 | 0:03:48 | 5.70% | 0.11% | 87.57% | 4.87% | 1.32% |
| 51 | 1:2 tetraglycol:Pr glycol | 12.1 | 0:03:10 | 0:03:23 | 5.66% | 0.03% | 91.23% | 2.30% | 0.36% |

TABLE II

| example | solvent | gIPN | 50 psi drop 1 (min) | 50 psi drop 2 (min) | % yield | | | | |
|---|---|---|---|---|---|---|---|---|---|
| | | | | | bicyclic | IPAN1 | IPDA | IPAA | amide |
| 10 | MeOH | 12.1 | 0:02:07 | 0:01:59 | 6.48% | 1.42% | 72.08% | 16.77% | 1.66% |
| 11 | EtOH | 12.0 | 0:03:28 | 0:03:53 | 4.32% | 0.00% | 78.48% | 8.45% | 8.21% |
| 12 | EtOH - run 2 | 12.1 | 0:02:35 | 0:03:05 | 4.53% | 0.00% | 79.47% | 14.05% | 1.52% |
| 13 | EtOH - run 3 | 12.1 | 0:04:04 | 0:02:51 | 4.57% | 0.00% | 78.22 | 15.44% | 1.35% |
| 14 | EtOH - run 4, prestir 2 hr at 60°C | 12.2 | 0:02:30 | 0:02:34 | 5.25% | 0.00% | 86.18% | 6.93% | 1.17% |
| 15 | EtOH - run 5, stir 1 hr w/Amberlyst 15 | 12.1 | 0:01:52 | 0:02:01 | 5.22% | 0.00% | 77.83% | 5.73% | 0.29% |
| 16 | t-BuOH | 12.2 | 0:06:16 | 0:06:39 | 3.34% | 0.16% | 62.85% | 18.00% | 14.93% |
| 17 | t-BuOH - run 2, prestir at 60°C | 12.3 | 0:03:38 | 0:04:36 | 4.93% | 0.16% | 77.15% | 12.05% | 5.13% |
| 18 | t-BuOH - run 3, stir 1 hr w/NH3 treated Amberlyst 15 | 12.3 | 0:03:25 | 0:04:25 | 5.08% | 0.85% | 81.37% | 8.90% | 3.32% |
| 19 | Me Carbitol | 12.2 | 0:05:36 | 0:05:02 | 3.61% | 0.08% | 69.27% | 18.03% | 8.32% |
| 20 | Me Carbitol run 2 | 12.2 | 0:04:13 | 0:05:21 | 3.47% | 0.04% | 68.92% | 26.25% | 0.96% |
| 21 | THF | 12.0 | 01:11:43 | 0:17:47 | 2.96% | 0.33% | 53.51% | 23.08% | 19.22% |
| 22 | THF - run 2 | 12.1 | 0:03:48 | 0:03:48 | 3.19% | 0.00% | 57.24% | 31.96% | 7.14% |

TABLE II

| example | solvent | gIPN | 50 psi drop 1 (min) | 50 psi drop 2 (min) | % yield bicyclic | IPANI | IPDA | IPAA | amide |
|---|---|---|---|---|---|---|---|---|---|
| 23 | pyridine | 12.0 | 0:15:10 | 0:14:58 | 2.59% | 0.14% | 58.56% | 18.83% | 18.70% |
| 24 | pyridine - run 2 | 12.0 | 0:10:53 | 0:09:22 | 3.33% | 0.10% | 66.06% | 21.31% | 8.04% |
| 25 | Methyl Propasol | 12.1 | 0:05:49 | 0:04:58 | 2.56% | 0.42% | 72.13% | 20.04% | 4.65% |
| 26 | Methyl Propasol-run 2 | 12.3 | 0:03:54 | 0:03:35 | 2.61% | 0.10% | 63.77% | 31.68% | 1.55% |
| 27 | Et glycol - run 1 | 12.2 | 0:04:33 | 0:04:55 | 6.00% | 0.27% | 88.58% | 3.62% | 0.60% |
| 28 | * run 2 | 12.1 | 0:04:32 | 0:05:55 | 5.09% | 0.30% | 87.87% | 5.35% | 0.68% |
| 29 | * run 3 | 12.1 | 0.05.53 | 0.04.26 | 5.10% | 0.39% | 87.67% | 5.44% | 0.69% |
| 30 | propylene glycol | 11.9 | 0.04:47 | 0:09:27 | 6.42% | 0.32% | 88.31% | 3.77% | 0.52% |
| 31 | propylene glycol - run 2 | 12.1 | 0.02:42 | 0:02:49 | 5.64% | 0.52% | 87.89% | 4.64% | 0.47% |
| 32 | 1,3 propandiol | 12.2 | 0:03:21 | 0:03:39 | 5.02% | 0.18% | 90.45% | 2.65% | 1.15% |
| 33 | 1,2 butanediol | 12.1 | 0:03:47 | 0:03:59 | 6.25% | 0.11% | 89.10% | 3.40% | 0.71% |
| 34 | 1,3 butanediol | 12.0 | 0:04:05 | 0:04:00 | 4.65% | 0.11% | 88.45% | 3.06% | 2.92% |
| 35 | 1,4 butanediol | 12.0 | leak | leak | 4.59% | 0.22% | 88.99% | 3.00% | 2.63% |
| 36 | tetraethylene glycol | 12.1 | 0:07:28 | 0:06:06 | 4.23% | 0.28% | 86.00% | 5.48% | 3.20% |
| 37 | Hexylene glycol | 12.1 | 0:08:07 | 0:09:42 | 4.49% | 0.00% | 75.07% | 9.72% | 10.00% |

Examples 52 to 54

In each of the examples, a five gram charge of chromium promoted Raney cobalt (Raney 2724) is used in a 100 milliliter Parr minireactor under the substantially same procedures as set forth for Examples 10 to

51. Methanol is used as the solvent for the reaction. The isophoronenitrile is introduced into the reactor as set forth in connection with Examples 10 to 51. The hydrogen (total pressure) is set forth in Table III. Each catalyst is subjected to a number of sequential runs to ascertain the changes in catalyst performance. The temperature programing is as set forth for Examples 10 to 51. Between runs, about 25 milliliters of the reaction menstruum are withdrawn from the minireactor, leaving about 10 milliliters of liquid slurried with the catalyst in the bottom of the reactor. The catalyst is not washed between runs. A 25 milliliter charge of fresh, make-up reaction menstruum containing isophoronenitrile is added for the subsequent run. Each run is about four hours in duration. The examples are summarized in Table III.

TABLE III

| Example | (Run) | Total Pressure, Psig | Initial rate (psi $H_2$/min) | Comments |
|---|---|---|---|---|
| 52 | (1) | 600 | 15.2 | |
| | (2) | 600 | 9.1 | |
| | (3) | 600 | 18.8 | The catalyst is subjected to a regeneration at 800 psig hydrogen at 80° C for 15 hours between runs 2 and 3. |
| | (4) | 600 | 6.2 | |
| 53 | (1) | 600 | 25.4 | |
| | (2) | 600 | less than 2 | The catalyst is subjected to 200 psig hydrogen at 120° C for 1 hour between runs 1 and 2. |
| | (3) | 600 | 17.7 | The catalyst is subjected to a regeneration at 800 psig hydrogen at 80° C for 15 hours between runs 2 and 3. |
| 54 | (1) | 1800 | 59.4 | |
| | (2) | 1800 | 51.3 | |
| | (3) | 1800 | 44.4 | |
| | (4) | 1800 | 23.0 | |
| | (5) | 500 | 10.4 | |

The examples indicate that at lower hydrogen partial pressures, the system containing methanol as the solvent tends to deactivate. At higher hydrogen pressures, as are typically disclosed in prior processes for the amination of, e.g., isophoronenitrile, the catalyst in methanol solvent tends to maintain its activity. However, the higher pressures also tend to result in the production of a greater proportion of the less desired aminoalcohol product.

Example 55

A slurry of 4.84 grams of chromium-promoted Raney cobalt (Raney 2724) in water is charged to a 100 milliliter stainless steel autoclave along with 35 milliliters of hydrogen-sparged solvent mixture of 85 volume per cent ethanol and 15 volume per cent isopropanol. The reactor is purged with hydrogen and the mixture stirred for several minutes. After allowing the catalyst to settle, the solvent is removed via a dip tube positioned about 7 millimeters above the reactor bottom. About 10 milliliters of solvent remain in the catalyst slurry. The catalyst is similarly washed with a second 35 milliliter aliquat of the hydrogen-sparged solvent.

A solution of 75 grams of isophoronenitrile in 75 milliliters of the ethanol/isopropanol solvent and the 4 grams of triethylene glycol dimethyl ether internal standard is charged to an 8 ounce glass pressure bottle containing a stir bar and attached to a gas manifold. The apparatus is evacuated, heated to 40° C in an oil bath and then pressurized to 40 psig with ammonia. After stirring under these conditions for 15 hours to form the imine, about 40 volume percent of this mixture is charged into the 100 milliliter autoclave containing the washed Raney cobalt catalyst. The reactor is heated to 60° C with rapid stirring and then pressurized to 525 psig with hydrogen. Rates were monitored by timing 50 psi pressure drops; the reactor is repressurized to 525 psig after each measurement. After 80 minutes under these conditions, the

temperature is increased at a rate of 20° C every 20 minutes to 120° C, then to 130° C after a further 10 minute period. The mixture is further stirred under 500 psig hydrogen for 1 hour. The product is removed via the dip tube after cooling and allowing the catalyst to settle. Subsequent runs are conducted the same way, except that the catalyst from the previous run is reused instead of using fresh catalyst. Table IV summarizes the results of a run.

## Example 56

A solution of 40.0 grams of isophoronenitrile and 22.01 grams of isophoronediamine in a mixture of 100 milliliters ethanol and 100 milliliters hexane is refluxed at ambient pressure to form an imine. Water is removed as the ternary azeotrope using a Dean-Stark trap. After refluxing for 9 hours (kettle temperature 62° C), azeotrope evolution ceased. A total of 53 milliliters of a lower azeotrope is collected, which consisted of 8.3 weight percent (4.4 grams) water by Karl Fisher analysis. The remaining solvent in the kettle is removed by rotary evaporation to yield 62.0 grams of a viscous liquid which is redissolved in an 85 volume percent ethanol and 15 volume percent isopropanol solvent to provide 98 grams of a two-phase solution. The solution is stirred under 40 psig ammonia at 40° C for 16 hours to yield a light yellow solution (total ammonia uptake 12.88 grams).

About 75 volume percent of this mixture is charged to a reactor containing 4.84 grams of Raney cobalt (Raney 2724) and about 10 milliliters of the final product from Example 55. The reaction procedure described in Example 55 is substantially followed.

Very few heavy materials that might have been generated by hydrogenation of imine formed from isophoronenitrile and isophoronediamine are found to be present. The results are summarized in Table IV.

Table IV

| | | | | | Weight % Yield | | | |
|---|---|---|---|---|---|---|---|---|
| Example | Isophorone-Nitrile, Grams | 50 psi Drop 1 (min;sec) | 50 psi Drop 2 (min;sec) | bicyclic* | IPANI* | IPDA* | IPAA* | Amide* |
| 55 | 30 | 0:00:51 | 0:01:04 | 7.46% | 0.11% | 84.01% | 4.30% | 0.26% |
| 56 | 30 | 0:30:55 | 0:05:28 | 5.19% | 1.02% | 88.38% | 1.00% | 0.26% |

*as defined in the previous examples

## Claims

1. A process for aminating a feed comprising organic compound containing at least one carbonyl group in a liquid menstruum comprising subjecting the feed to reductive amination conditions comprising the presence of hydrogen and at least one of ammonia, primary amine and secondary amine and the presence of reductive amination catalyst to convert at least one carbonyl group of the organic compound in a portion of the feed to an amino group to provide an amine product and to convert at least one carbonyl group of the organic compound in another portion of the feed to a hydroxyl group to provide a hydroxylated product wherein the liquid menstruum comprises a sufficient amount of at least one amination promoter to enhance selectivity to the amine product as compared to the hydroxylated product.

2. The process of claim 1 wherein the reductive amination catalyst comprises at least one of Raney nickel catalyst and Raney cobalt catalyst.

3. The process of claim 1 or 2 wherein a primary amine is present in the liquid menstruum which primary amine is capable of reacting with the carbonyl group of the organic compound to generate an imino group.

4. The process of claim 1 wherein the reductive amination is conducted in two steps: (a) subjecting the organic compound containing at least one carbonyl group under imine-forming conditions to convert at least one carbonyl group of the organic compound in a portion of the feed to an imino group to produce an imine product in the presence of at least one amination promoter, and (b) subjecting the imine product under

amine-forming conditions to convert at least one imino group of at least a portion of the imine product to an amino group to provide an amine product.

5. The process of claim 4 wherein at least during a portion of step (a) the hydrogen partial pressure is at least 6,9 bar (100 psi) lower than the hydrogen partial pressure during at least a portion of step (b) and ammonia is present at least during a portion of step (b).

6. The process of anyone of claims 1 to 5 wherein the reductive amination conditions comprise a temperature between about 10° and 300° C and a hydrogen partial pressure of less than about 69 bar (1000 psi).

7. A process for aminating a feed comprising organic compound containing at least one carbonyl group in a liquid menstruum comprising (a) contacting the feed with at least one of ammonia, primary amine and secondary amine under imine-forming conditions to convert at least one carbonyl group of the organic compound in at least a portion of the feed to an imino or enamino group to provide an organic compound containing at least one imino or enamino group and (b) contacting at least a portion of the organic compound containing at least one imino group with hydrogen under amine-forming conditions to convert at least one imino group of at least a portion of the organic compound to an amine group to produce an amine product, wherein during at least a portion of step (a) the hydrogen partial pressure is at least 6,9 bar (100 psi) lower than the hydrogen partial pressure during at least a portion of step (b).

8. The process of claim 7 wherein in step (a) primary amine is present.

9. The process of claim 7 or 8 wherein at least one amination promoter is present in the liquid menstruum during at least a portion of step (a).

10. The process of anyone of claims 1 to 9 wherein the amination promoter comprises dipolar protic compound having preferably more than one carbon atom and/or has a $pK_a$ at 25° C in a 0.4 to 1 weight per cent solution in dimethylsulfoxide of less than about 35 and preferably has a dielectric constant at 25° C greater than about 8.

11. The process of anyone of claims 1 to 10 wherein the at least one amination promoter has at least one hydroxyl group and preferably a molecular weight of less than about 200, and preferably contains at least two hydroxyl groups, and especially comprises at least one of ethylene glycol, 1,2-propylene glycol, 1,3-propanediol, 1,2-butanediol, 1,3-butanediol, 1,4-butanediol, glycerol, diethylene glycol, triethylene glycol, tetraethylene glycol and sorbitol.

12. A process for aminating a feed comprising organic compound containing at least one carbonyl group in a liquid menstruum comprising (a) contacting the feed with at least one of primary amine and secondary amine under imine-forming conditions to convert at least one carbonyl group of the organic compound in at least a portion of the feed to an imino or enamino group to provide an organic compound containing at least one imino or enamino group and (b) contacting at least a portion of the organic compound containing at least one imino or enamino group with hydrogen under amine-forming conditions to convert at least one imino or enamino group of at least a portion of the organic compound to an amine group to produce an amine product.

13. The process of claim 12 wherein the amine-forming conditions include the presence of at least one reductive amination catalyst, preferably ammonia.

14. The process of anyone of claims 1 to 13 wherein the primary amine is represented by the formula $RNH_2$ wherein R is alkyl, hydroxy, hydroxyalkyl, aminoalkyl, aryl, hydroxyaryl, amino, or aminoaryl of up to 20 carbon atoms.

15. The process of claim 14 wherein the primary amine comprises an amine the same as the amine product.

16. The process of anyone of claims 4 to 15 wherein at least one amination promoter is present in the liquid menstruum during at least a portion of step (a).

17. The process of anyone of claims 1 to 16 wherein the hydrogen partial pressure is less than 48,3 bar (700 psi).

18. The process of any one of claims 1 to 17 wherein the organic compound containing at least one carbonyl group has the formula $R^1C(O)R^2$ wherein $R^1$ is hydrogen or a substituent represented by $R^2$ and wherein $R^2$ is hydrocarbyl or substituted hydrocarbyl of 1 to 30 carbon atoms.

19. A process £or aminating a feed comprising organic compound containing at least one carbonyl group in a liquid menstruum comprising subjecting the feed to imine-forming conditions comprising the presence of at least one of primary amine and secondary amine to convert at least one carbonyl group of the organic compound in a portion of the feed to an imino or enamino group to provide an imino or enamino product and water, removing water from the liquid menstruum containing the imino or enamino product, and subjecting the imino or enamino product to reductive amination conditions including the presence of hydrogen, sufficient ammonia to reequilibrate the imino or enamino product and reductive amination catalyst

to convert at least one imino or enamino group of at least a portion of the imino or enamino product to primary amine to provide an amine product, wherein at least one carbonyl group of the organic compound in another portion of the feed is converted to a hydroxyl group to produce a hydroxylated product, and wherein sufficient water is removed to enhance selectivity to the amine product as compared to the hydroxylated product.

20. The process of claim 19 wherein the water is removed by at least one of distillation, phase separation, sorption and membrane permeation, or wherein water is sorbed with a liquid or solid sorbent contained in the reaction medium during the reductive amination.

21. The process of anyone of claims 1 to 20 wherein the organic compound containing at least one carbonyl group has the formula $R^1C(O)R^2$ wherein $R^1$ is hydrogen or a substituent represented by $R^2$ and wherein $R^2$ is hydrocarbyl or substituted hydrocarbyl of 1 to 30 carbon atoms.

22. The process of claim 21 wherein the reductive amination is conducted in two steps: (a) subjecting in the presence of at least one amination promoter, the organic compound containing at least one carbonyl group under imine-forming conditions to convert at least one carbonyl group of the organic compound in a portion of the feed to an imino group to produce an imine product, and (b) subjecting the imine product under amine-forming conditions to convert at least one imino group of a portion of the imine product to an amino group to provide the amine product.

23 . The process of anyone of claims 4 to 22 wherein a portion of step (a) is conducted simultaneously with at least a portion of step (b).

24. The process of claim 23 wherein at least a portion of the water removed is removed during step (a).

25. The process of anyone of claims 4 to 24 wherein at least one amination promoter is present in the liquid menstruum during at least a portion of step (b).

26. A process for activating a reductive amination catalyst used for aminating organic compound containing at least one carbonyl group in a liquid menstruum comprising contacting the liquid menstruum with hydrogen and at least one of ammonia, primary amine, and secondary amine under reductive amination conditions including the presence of the reductive amination catalyst to convert at least one carbonyl group of at least a portion of the organic compound to an amine group, said activation comprising contacting the reductive amination catalyst with hydrogen at a partial pressure and temperature for a time sufficient to increase the activity of the catalyst.

27. The process of claim 26 wherein the catalyst is a catalyst having been deactivated by reductive amination at a hydrogen partial pressure of less than about 103,5 bar (1500 psi).

28. The process of claim 27 wherein the hydrogen partial pressure for reductive amination is less than about

29. The process of anyone of claims 27 to 28 wherein the partial pressure of hydrogen for the activation is at least about 500 psi and the temperature is at least about 40 °C.

30. The process of anyone of claims 26 to 29 wherein the hydrogen for activation has an essential absence of ammonia and amine.

European Patent Office

# EUROPEAN SEARCH REPORT

Application Number

EP 90 10 7780

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 011 656 (CHEMISCHE WERKE HUELS AG)<br>* the whole document *<br>--- | 1-4,6, 12,13, 18-20 | C 07 C 209/26<br>C 07 C 209/52 |
| A | EP-A-0 042 119 (CHEMISCHE WERKE HUELS AG)<br>* the whole document * & US-4429157 (Cat. D)<br>--- | 1-4,6, 13,17-22 | |
| A | EP-A-0 010 179 (RUHRCHEMIE)<br>* page 1-7; claims 1,2 *<br>--- | 1-6 | |
| A | EP-A-0 014 985 (UNIROYAL)<br>* the whole document *<br>--- | 1,2,10, 11 | |
| A,D | US-A-3 352 913 (KARL SCHMITT et al.)<br>* the whole document *<br>--- | 1-7 | |
| A,D | PATENT ABSTRACTS OF JAPAN<br>vol. 11, no. 353 (C-457)(2800), 18 November 1987; & JP-A-62123154 (DAICEL CHEM IND LTD.) 04.06.1987<br>--- | 1-7 | **TECHNICAL FIELDS SEARCHED (Int. Cl.5)** |
| A | EP-A-0 093 892 (HOECHST AG)<br>* the whole document *<br>----- | 1-4,6, 10,11 | C 07 C 209/00 |

The present search report has been drawn up for all claims

| Place of search | Date of completion of the search | Examiner |
|---|---|---|
| BERLIN | 25-06-1990 | RUFET J.M.A. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document